# EUROPÄISCHE PATENTSCHRIFT

(11) **EP 3 209 224 B1**
(45) Veröffentlichungstag und Bekanntmachung des Hinweises auf die Patenterteilung: **09.12.2020**
(21) Anmeldenummer: 15808318.8
(22) Anmeldetag: 16.10.2015
(51) Int. Cl.: A61B 17/20, A61B 10/00

(54) **BLISTERSTREIFEN**
BLISTER STRIP
BANDE ALVÉOLAIRE

(30) Priorität: 21.10.2014 AT 507552014
(43) Veröffentlichungstag der Anmeldung: 30.08.2017
(73) Patentinhaber: ALLTEST GmbH, 4020 Linz (AT)
(72) Erfinder: FORSTNER, Bernhard, 4020 Linz (AT)
(74) Vertreter: Burgstaller, Peter
(86) Internationale Anmeldenummer: PCT/AT2015/050258
(87) Internationale Veröffentlichungsnummer: WO 2016/061600

(56) Entgegenhaltungen:
- EP-A1- 0 734 964
- WO-A1-88/09149
- WO-A1-96/32142
- US-A- 2 841 138
- US-A- 4 205 689
- US-A- 5 104 620
- US-A- 5 179 959

## Beschreibung

Die Erfindung betrifft einen Blisterstreifen zum Aufkleben auf die Haut, insbesondere zur Durchführung eines Allergietests.

Ein Blisterstreifen ist ein Schichtverbund, welcher zumindest zwei Schichten umfasst, wobei zwischen den Schichten ein oder mehrere Hohlräume liegen, wobei die Hohlräume als Blister bezeichnet werden. Blisterstreifen sind beispielsweise als Medikamentenverpackung bekannt, wobei in den Blistern Wirkstoffe in Form von Pillen enthalten sind, welche aus dem Blister herausgedrückt werden können. Ein Blisterstreifen besteht dabei aus zwei Folien, wobei die untere Folie flach ist und die obere Folie kuppelförmige formstabile Ausstülpungen (Beulen, Blister) aufweist, sodass sich zwischen den Folien Hohlräume bilden. Durch Aufbringen von Druck auf die Kuppel der oberen Folie reißt die untere Folie ein und der Inhalt des Hohlraums tritt aus.

Zum Durchführen von Allergietest sind auf die Haut aufbringbare Teststreifen bekannt, welche in einer Kapsel oder einem Hohlraum ein Allergen enthalten, welches zumeist in einer flüssigen oder gelartigen Trägersubstanz vorliegt. Bei diesen Allergietests ist durch Öffnen oder Zerstören der Kapsel bzw. des Hohlraums das Allergen mit der Haut in Kontakt bringbar. Es ist nach dem Stand der Technik bekannt diese Allergieteststreifen als Blisterstreifen auszuführen. Nach dem Stand der Technik sind auch Blisterstreifen bekannt, welche auf die Haut der zu testenden Person aufklebbar sind.

Bei den aufklebbaren Blisterstreifen, welche flüssige Allergene beinhalten, ist es bekannt, diese gemäß dem oben beschriebenen Aufbau der Medikamentenverpackung herzustellen. Bei Anwendung wird die untere Folie auf die Haut aufgeklebt. An der Innenseite der Kuppel der oberen Folie ist ein Applikator angebracht, welcher in der Lage ist die untere Folie zu durchstechen und ggf. auch geringfügig in die Haut einzudringen. Die Kuppel ist zumindest teilweise mit Flüssigkeit gefüllt, wobei diese durch das Loch in der Folie austritt und so auf bzw. in die Haut gelangen soll. Blisterstreifen nach diesem Prinzip sind beispielsweise in der WO 8705200 A1 und der US 2014276196 A1 gezeigt. Nachteilig an diesem Aufbau ist, dass mit dem Applikator die Folie durchstochen werden muss, was ein unregelmäßiges, bzw. unzuverlässiges Ausbringen des Allergens zur Folge haben kann, bzw. das Risiko birgt, dass Teile der Folie in die Haut eindringen. Nachteilig ist, dass dieser Aufbau nur eine nahezu punktförmige Einbringung des Allergens ermöglicht und ein Verfließen der Flüssigkeit zwischen Haut und Folie mit der Gefahr der Vermischung möglich ist.

Bei anderen aufklebbaren Blisterstreifen ist es bekannt, das Allergen als Gel vorzusehen, oder in Flüssigkeit in einem saugfähigen Substrat oder die Flüssigkeit durch Oberflächenspannung an der Innenseite des Blisters zu halten. Blisterstreifen mit diesem Prinzip werden beispielsweise in der US4802493A, der US4966159A und der US2007276284A1 gezeigt. In diesem Fall kann die untere Folie vor dem Anbringen des Blisterstreifens entfernt werden, ohne dass Flüssigkeit austritt. An der Innenseite der Kuppel der oberen Folie kann wiederum ein Applikator vorhanden sein, mit welchem die Haut in geringem Maß verletzt werden kann. Nachteilig ist, dass das Allergen in saugfähigem Substrat oder als Gel vorliegen muss, zudem besteht die Gefahr, dass nach Abziehen der Folie beim Hantieren das Allergen an einer falschen Stelle aufgebracht wird oder das Gel bzw. das saugfähige Substrat mit anderen Allergenen (beispielsweise aus andern Blistern) verunreinigt wird.

Ferner ist in der US 5099857 A gezeigt, im Blister unterhalb des Applikators eine zusätzliche Kapsel vorzusehen, welche zur Freisetzung der Testflüssigkeit zerstört wird.

Die US 2841138 A zeigt eine Folie, auf welcher mehrere Applikatoren nebeneinander aufgebracht sind.

Die der Erfindung zu Grunde liegende Aufgabe besteht darin, einen aufklebbaren Blisterstreifen für Allergietests bereit zu stellen, welcher einen einfachen Aufbau aufweist und welcher den Einsatz von flüssigen Testsubstanzen ermöglicht, wobei im Blister ein Applikator angebracht sein soll, welcher eine kontrollierte geringfügige Verletzung der Haut ermöglicht, wobei bei der Anwendung vom Applikator keine Folie durchstochen werden soll.

Für das Lösen der Aufgabe wird vorgeschlagen, den Applikator derart auszugestalten bzw. im Blister anzuordnen, dass dieser den Blister in zwei Bereiche trennt. Der erste Bereich liegt zwischen der Innenseite der bevorzugt kuppelförmigen Ausstülpung der oberen Folie und dem Applikator und wird in Folge als Flüssigkeitsreservoir bezeichnet. Der zweite Bereich liegt zwischen dem Applikator und der unteren Folie und wird in Folge als Auspressreservoir bezeichnet. Durch den Applikator sind die beiden Bereiche im ungeöffneten Zustand des Blisterstreifens dicht voneinander getrennt.

Um den Blisterstreifen zu applizieren wird die untere Folie vom Streifen abgezogen und so die Unterseite der oberen Folie freigelegt, welche zumindest im Bereich rund um jeden Blister mit einem hautverträglichen Klebstoff versehen ist. Nun wird die Unterseite der oberen Folie auf die Haut aufgeklebt, sodass das Auspressreservoir nun durch den Applikator, gegebenenfalls durch die Seitenwände der Ausstülpung unterhalb des Applikators und die Haut gebildet ist. Durch Ausüben einer Kraft auf die Kuppel des Blisters wird die Flüssigkeit des Flüssigkeitsreservoirs durch eine Öffnung im Applikator ins Auspressreservoir bewegt.

Wird nach dem Auspressen des Flüssigkeitsreservoirs die Kraft auf den Applikator noch etwas erhöht, wird dieser in Richtung der Haut bewegt und es kann beispielsweise durch Fingerdruck und vorsichtige kreisende, massierende Bewegung des Applikators eine oberflächliche Verletzung der Haut in Form von Kratzern erreicht werden und das Eindringen der Allergenflüssigkeit in die obersten Hautschichten erreicht werden.

Danach wird die Krafteinwirkung weggenommen und der Blisterstreifen für einige Minuten auf der Haut belassen um eine Reaktion des Körpers auf das Allergen abzuwarten, bevor der Blisterstreifen abgezogen wird.

Die Erfindung wird an Hand von Zeichnungen veranschaulicht:
- Fig. 1:: zeigt in Schnittansicht den Aufbau eines beispielhaften erfindungsgemäßen Blisterstreifens.
- Fig. 2:: zeigt in Schnittansicht einen beispielhaften erfindungsgemäßen Blisterstreifen nach Entfernen der unteren Folie.
- Fig. 3:: zeigt in Schnittansicht einen beispielhaften erfindungsgemäßen Blisterstreifen, welcher auf die Haut aufgeklebt ist mit einem Blister Applikator im Ausgangszustand und einem Applikator, der bereits eingedrückt wurde und bei dem so die Allergenflüssigkeit in das Auspressreservoir ausgepresst wurde.
- Fig. 4:: zeigt in Schnittansicht einen beispielhaften erfindungsgemäßen Blisterstreifen, welcher auf die Haut aufgeklebt ist mit druckbetätigtem Applikator und erfolgter Hautpenetration.
- Fig. 5:: zeigt in Schnittansicht einen beispielhaften erfindungsgemäßen Blisterstreifen mit alternativen Ausgestaltungen.
- Fig. 6:: zeigt einen beispielhaften erfindungsgemäßen Blisterstreifen von oben.
- Fig. 7:: die Fig. 7a bis 7d zeigen in Schnittansicht einen beispielhaften erfindungsgemäßen Blisterstreifen in mehreren Schritten eines beispielhaften Herstellungsverfahrens.
- Fig. 8:: die Fig. 8a bis 8c zeigen beispielhafte erfindungsgemäße Applikatorspitzen.

Soweit in der Figurenbeschreibung die Richtungsangaben oben und unten bzw. obere und untere verwendet werden, bezieht sich dies auf die Lage des Blisterstreifens in Ausrichtung zu jener Fläche auf die der Streifen bei Applikation bestimmungsgemäß aufgebracht wird. Unten bzw. die Unterseite ist also jene Seite die besagter Fläche zugewandt liegt.

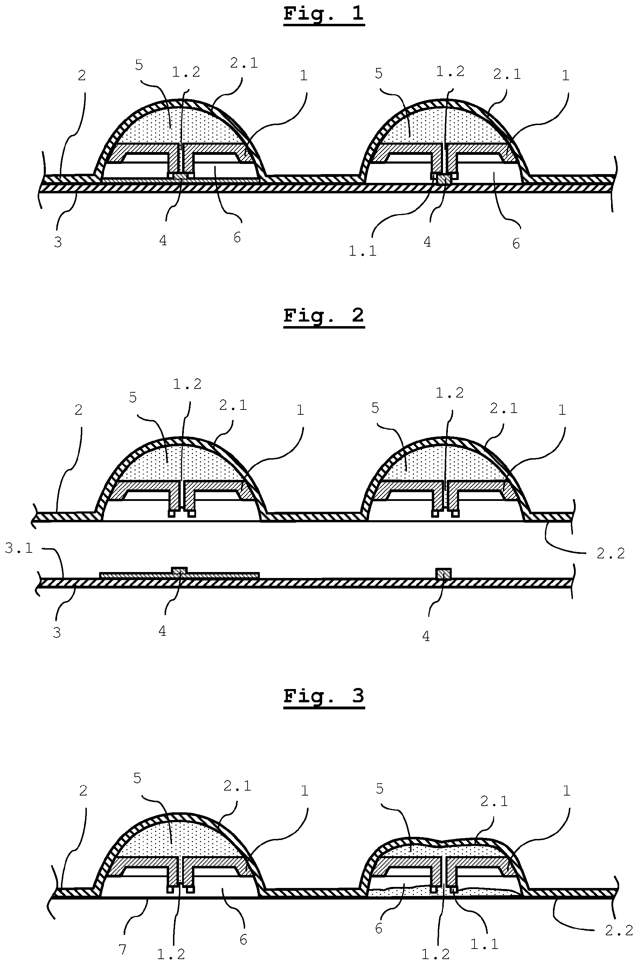

In Fig. 1 ist der Aufbau eines bevorzugten erfindungsgemäßen Blisterstreifens gezeigt, wobei zwei Blister im Querschnitt gezeigt sind, wobei jeder Blister durch eine Ausstülpung 2.1 der oberen Folie 2 gebildet ist, welche wie dargestellt bevorzugt eine Kuppelform aufweist. In jedem Blister ist ein Applikator 1 angebracht, welcher an der ringförmigen Berührungsfläche mit der Innenseite der Ausstülpung 2.1 verklebt, oder flüssigkeitsdicht verpresst ist. Die Ausstülpung 2.1 ist so durch den Applikator 1 in zwei Bereiche geteilt. Der Applikator 1 weist eine Öffnung 1.2 auf, durch welche bei der Applikation des Blisterstreifens Flüssigkeit treten kann.

Die beiden Bereiche sind im ungeöffneten Zustand des Blisterstreifens dicht voneinander getrennt, was beispielsweise durch einen Dichtungspfropfen 4 erreicht werden kann, welcher durch Klebung mit der unteren Folie 3 verbunden ist und so mit dieser entfernt werden kann. Dieser Dichtungspfropfen 4 kann beispielsweise nur die Öffnung 1.2 verschließen, oder wie im linken Blister dargestellt die gesamte Grundfläche des Blisters. Diese Ausführung hat den Vorteil, dass der Dichtungspfropfen 4 leicht maschinell einsetzbar ist und bei der Fertigung des Blisterstreifens kein Klebstoff in den Blister, bzw. an die Applikatorspitze 1.1 gelangen kann.

Der Applikator 1 weist bevorzugt eine Scheibenform auf, wobei der Rand der Scheibe etwas verbreitert ist und nach unten von der Scheibe vorragt, um die Kontaktfläche mit der Ausstülpung 2.1 zu erhöhen. Zentral steht aus dem Applikator 1 die Applikatorspitze 1.1 ebenfalls nach unten vor, wobei die Applikatorspitze 1.1 bevorzugt weiter nach unten vorragt als der Rand der Scheibe, sodass diese beim Hinunterdrücken des Applikators 1 vor dem Rand der Scheibe mit der Haut in Kontakt kommt, andernfalls müsste der Applikator 1 selbst etwas verformbar, bevorzugt elastisch verformbar sein, sodass dessen Zentrum und somit die Applikatorspitze 1.1 etwas nach unten bewegt werden kann, wenn der Rand der Scheibe bereits in Kontakt mit der Haut 7 ist. Die Scheibe ist bevorzugt so eingesetzt, dass unterhalb der Scheibe die Kuppelwände der Ausstülpung 2.1 einen Begrenzung des Auspressreservoirs 6 bilden, also des untere Ende des Rands der Scheibe beabstandet zur Unterseite 2.2 der Folie 2 in die Ausstülpung 2.1 eingesetzt ist. Wenn die Ausstülpung 2.1 eine Kuppelform aufweist, ist die Fläche, mit welcher die Scheibe an der Ausstülpung 2.1 anliegt, bevorzugt an die Form der Kuppel angepasst, also ringförmig und nach oben hin verjüngend ausgeführt, sodass die Scheibe in etwa eine Kegelscheibe oder Kugelscheibe ist.

Die Applikatorspitze 1.1 ist jener Teil des Applikators 1, welcher mit der Haut 7 in Kontakt bringbar ist um eine geringfügige Läsion dieser verursachen zu können. Die Applikatorspitze 1.1 weist dabei eine oder mehrere scharfe oder spitze Elemente oder Kanten auf, mit welchen die Haut 7 gekratzt bzw. geritzt oder punktuell penetriert werden kann. Die Applikatorspitze 1.1 kann dabei eine Hohlnadelspitze oder Pricknadelspitze sein oder mehrere dieser aufweisen, oder ähnlich wie Schleifpapier mehrere regelmäßig oder unregelmäßig von der Oberfläche vorspringende Geometrien aufweisen. Die scharfen oder spitzen Elemente sind bevorzugt kranzförmig um die vorzugsweise zentral in der Applikatorspitze 1.1 verlaufende Öffnung 1.2 des Applikators 1 angebracht. Ist die Applikatorspitze 1.1 eine Hohlnadel, so kann die Öffnung der Hohlnadel die Öffnung 1.2 des Applikators 1 sein.

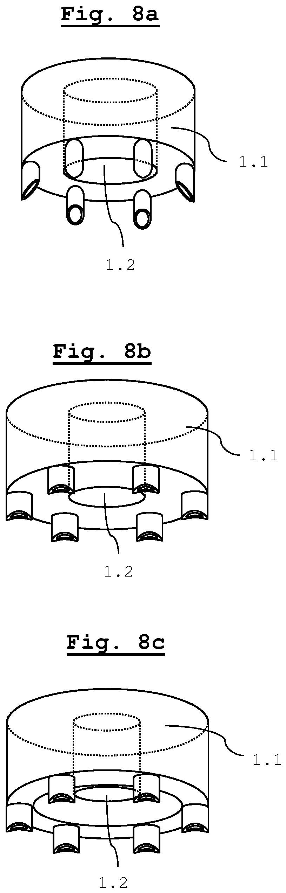

Drei beispielhafte besonders bevorzugte erfindungsgemäße Applikatorspitzen 1.1 sind in den Fig. 8a, 8b, 8c gezeigt. Die erfindungsgemäße Applikatorspitze wurde speziell für diese Anwendung erfunden, aufgrund der vorteilhaften Ausgestaltung kann diese aber auch für andere Anwendungen verwendet werden, beispielsweise zur vorteilhaften Verbesserung von bekannten (Allergietest-)Applikatoren. Die erfindungsgemäße Applikatorspitze 1.1 weist zumindest eine bevorzugt zentrale Öffnung 1.2 auf, und ist beispielsweise als Zylinder ausgeführt. An der der Haut 7 bzw. der unteren Folie 3 zugewandten Seite weist die Applikatorspitze 1.1 mehrere scharfe bzw. spitze Elemente auf, welche bevorzugt kranzförmig um die Öffnung 1.2 angeordnet sind. Besonders vorteilhaft sind diese Elemente durch Hohlnadelspitzen gebildet, welche bei Applikation eine kleine Menge Flüssigkeit aufnehmen können und diese in die Haut 7 einbringen können, bzw. nach Penetration der obersten Hautschichten durch Kontakt mit der Allergenflüssigkeit verzögert einbringen können. Wie in Fig. 8a gezeigt können konventionelle schräg abgeschnittene Hohlnadeln als spitze Elemente verwendet werden, wobei diese soweit in die Haut eindringen können, wie sie aus der Applikatorspitze 1.1 hervorragen.

Bevorzugt sind die Hohl- bzw. Penetratornadeln nicht schräg sondern horizontal abgesetzt wie in den Fig. 8b und 8c gezeigt, um eine tiefe Penetration zu vermeiden und somit wirklich nur die oberste Hautschicht zu penetrieren. Die Form der Penetratornadelspitze ist dabei besonders bevorzugt konkav, muldenförmig eingeformt und kann auch mit einer feinsten Zahnung am Umfang versehen sein.

Wie in Fig. 8c gezeigt kann die Applikatorspitze 1.1 selbst an der Unterseite bevorzugt muldenförmig bzw. konkav eingeformt sein, sodass im Bereich zwischen den spitzen, bzw. scharfen Elementen eine größere Menge an Flüssigkeit verbleiben kann. Dies kann beispielsweise dadurch erreicht werden, dass die kleine Öffnung 1.2 am unteren Ende eine Phase, Abrundung oder Senkung aufweist um deren Durchmesser zu vergrößern. Bevorzugt wird die Applikatorspitze 1.1 im Spritzgussverfahren gefertigt, wobei bevorzugt auch die spitzen bzw. scharfen Elemente und oder der Applikator 1 in der Spritzgussform ausgeformt sind, sodass in einem Arbeitsschritt ein fertig einsatzbereiter Applikator 1 gefertigt wird. Sofern die spitzen bzw. scharfen Elemente aus einem anderen Material wie die Applikatorspitze 1.1 bestehen, sind diese bevorzugt in die Spritzgussform eingesetzt, sodass diese beim Spritzguss teilweise ins Material der Applikatorspitze eingebettet werden. Wie in Fig. 2 dargestellt, kann die untere Folie 3 abgezogen werden, wobei die obere Fläche 3.1 der unteren Folie 3, welche an der Unterseite 2.2 der oberen Folie 2 anliegt, als Klebeschutzfolie ausgeführt ist. Damit der Dichtpfropfen 4 an der unteren Folie 3 anhaften, kann die untere Folie 3 im Bereich des Dichtungspfropfen 4 nicht als Klebeschutzfolie ausgeführt sein. Durch Abziehen der unteren Folie 3 mit dem Dichtungspfropfen 4 werden die klebende Unterseite 2.2 der oberen Folie 2 und die Öffnung 1.2 des Applikators 1 freigelegt. Die Flüssigkeit tritt, solange kein Druck auf die Ausstülpung 2.1 ausgeübt wird, nicht durch die Öffnung 1.2, da durch die kleine Öffnung 1.2 keine Luft in das Flüssigkeitsreservoir 5 eindringen kann.

Der Dichtungspfropfen 4 dient also dazu, während der Lagerung oder Handhabung des ungeöffneten Blisterstreifens zu verhindern, dass durch unbeabsichtigtes Zusammendrücken der Ausstülpung 2.1 das Flüssigkeitsreservoir 5 in das Auspressreservoir 6 entleert wird. Wie in Fig. 3 dargestellt wird der geöffnete Blisterstreifen mit der klebenden Unterseite 2.2 der oberen Folie 2 auf die Haut 7 aufgeklebt, wodurch zwischen Applikator 1 und der Haut 6 ein gegenüber der Umgebung abgedichteter Hohlraum in Form des Auspressreservoirs 6 gebildet ist. Wie im zweiten Blister der Fig. 3 zu erkennen ist, kann durch Zusammendrücken der Ausstülpung 2.1 das Volumen des Flüssigkeitsreservoirs 5 verringert werden, wodurch die Flüssigkeit durch die Öffnung 1.2 des Applikators 1 gedrückt wird und in das Auspressreservoir 6 gelangt.

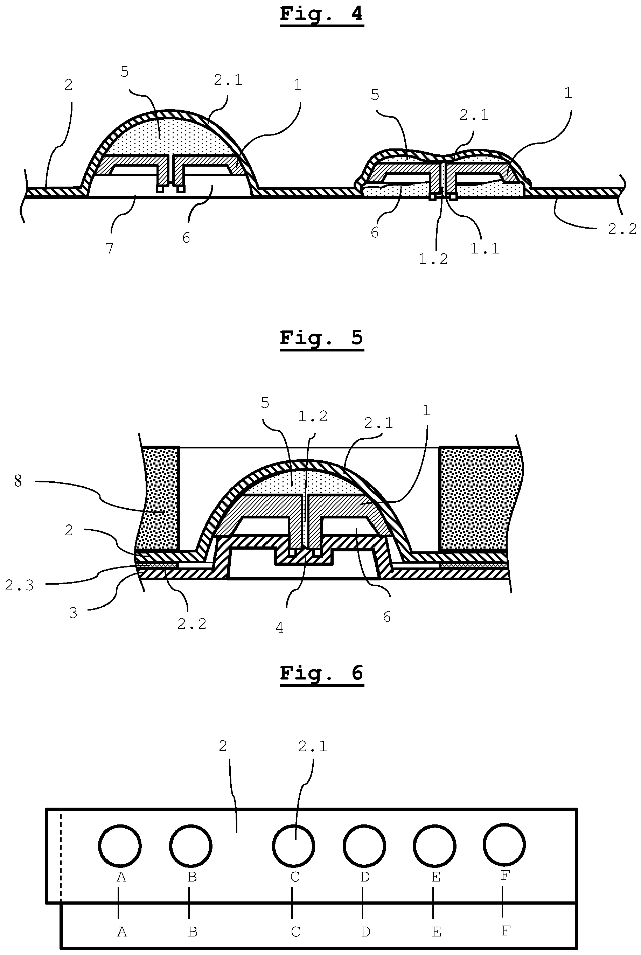

Wie beim zweiten Blister in Fig. 4 dargestellt kann durch weiteres Zusammendrücken der Ausstülpung 2.1 der Applikator 1 in Kontakt mit der Haut 7 gebracht werden. Dies erfolgt wenn die Innenseite der Ausstülpung 2.1 am Applikator 1 anliegt, beziehungsweise wenn der Widerstand der Flüssigkeit gegen das Auspressen höher ist als der Widerstand der Kuppelseitenwände, welche unterhalb des Applikators 1 das Auspressreservoir 6 seitlich begrenzen, gegen Verformung. Aufgrund der Kuppelgeometrie erfordert das Zusammendrücken der oberen flachen Kuppelkappe, welche das Flüssigkeitsreservoir 5 begrenzt, weniger Kraft als das Zusammendrücken der steilen Kuppelseitenwände unterhalb des Applikators 1.

Ist die Applikatorspitze 1.1 in Kontakt mit der Haut 7 kann durch leicht kreisendes Massieren des Blisters das Allergen in die Haut 7 eingebracht werden. Dazu dringen die scharfen bzw. spitzen Elemente der Applikatorspitze 1.1 etwas in die Haut 7 ein. Durch geeignete Ausgestaltung der Applikatorspitze 1.1, beispielsweise durch den Abstand mit welchem die scharfen oder spitzen Elemente aus der Applikatorspitze 1.1 hervorstehen, kann festgelegt werden wie tief bzw. in welche Hautschicht die Allergene eindringen. Der gegenständliche Aufbau ist auch vorteilhaft, wenn der Applikator 1 keine Spitze bzw. scharfe Elemente aufweist, beispielsweise zur Durchführung von Epikutantests.

Je nach Dimension des Blisters, bzw. je nach Menge an Flüssigkeit im Flüssigkeitsreservoir 5 kann es erforderlich sein, eine Möglichkeit zu schaffen, um die im Auspressreservoir 6 eingeschlossene Luft entweichen zu lassen, damit diese nicht unter die klebende Schicht der Folie 2 hineingepresst wird und dadurch möglicherweise ein ungewolltes, unkontrolliertes Entweichen von Flüssigkeit erfolgt. Eine Möglichkeit ist eine Sollbruchstelle vorzusehen, sodass Flüssigkeit nur seitlich unter der Klebeschicht entweichen kann und somit in die Umgebung gelangt und nicht in einen anderen Blister. Eine weitere Möglichkeit wäre das Auspressreservoir 6 mit einem expandierbaren Volumen zu verbinden, beispielsweise einem zweiten leeren zusammengedrückten Blister, oder einem weiteren zwischen der Ausstülpung 2.1 und dem Applikator 1 liegenden abgetrennten Volumen, welches im Ausgangszustand zusammengedrückt ist.

Wenn der Blister im Vergleich zu Klebefläche sehr kleinflächig ist (bzw. der Abstand zwischen zwei Blistern ausreichend groß ist), oder das Volumen des Flüssigkeitsreservoirs 5 im Vergleich zum Volumen des Auspressreservoirs 6 klein ist, kann auf das Vorsehen eines Luftauslasses verzichtet werden.

Bevorzugt beträgt das Volumen des Flüssigkeitsreservoirs 5 zirka ein Fünftel des Volumens des Auspressreservoirs 6. Das Volumen des Flüssigkeitsreservoirs 5 beträgt bevorzugt zwischen 20 und 30 µl. In diesem Fall ist das Vorsehen eines Luftauslasses nicht notwendig, da die geringe Volumenänderung des Auspressreservoirs 6 durch Einbringen der Flüssigkeit durch die elastische Nachgiebigkeit der Haut 7 ausgeglichen wird. Während der Applikation wird durch das Heranführen der Spitze 1.1 an die Haut 8 das Volumen des Auspressreservoirs 6 zudem etwas verringert, bzw. der geringe Überdruck im Auspressreservoir 6 etwas erhöht, welcher zu einer weiteren Wölbung der Haut 7 führt. Da der Allergie-Test üblicherweise am Unterarm oder am Rücken des liegenden Patienten durchgeführt wird, entsteht so in der Haut 7 eine kleine Mulde im Zentrum des Blisters, in der sich die Flüssigkeit sammelt.

Als Möglichkeit das zusätzliche Volumen aus dem Flüssigkeitsreservoir 5, bzw. das durch das Hinunterdrücken des Applikator 1 verringerte Volumen des Auspressreservoirs 6 auszugleichen, kann wie in Fig. 5 gezeigt der Klebeauftrag auf der Unterseite 2.2 der oberen Folie 2 etwas beabstandet zum Blister erfolgt. Der Klebeauftrag ist in diesem Fall durch eine doppelseitig klebende Folie 2.3 gebildet. Wie in Fig. 5 dargestellt kann der Blisterstreifen eine zusätzliche Schutzschicht 8 aufweisen, welche an der oberen Fläche der oberen Folie 2 aufliegt oder befestigt ist. Diese bevorzugt aus Karton oder geschäumten Kunststoff gebildete Schutzschicht 8 weist Ausnehmungen für die Blister auf und schützt die Blister bei Lagerung oder beim Aufkleben auf die Haut 7 vor Beschädigung. Die Applikation wird durch die Schutzschicht 8 nicht behindert, da der Blister durch die Öffnung in der Schutzschicht 8 zugänglich ist. In Fig. 5 ist zudem gezeigt, dass auch die untere Folie 3 im Bereich des Blisters tiefgezogen sein kann, sodass diese selbst den Dichtungspfropfen 4 bildet bzw. die Öffnung 1.2 und/oder die ganze Grundfläche des Blisters verschließt.

Als Material für den Applikator 1 eignet sich formstabiles Material wie Hartplastik, insbesondere transparentes Hartplastik. Der Applikator 1 wird besonders bevorzugt kostengünstig im Spritzgussverfahren gefertigt. Die scharfen bzw. spitzen Elemente der Applikatorspitze 1.1 können ebenfalls aus Hartplastik bestehen und mit dem Applikator 1 monolithisch verbunden sein. Die scharfen bzw. spitzen Elemente können auch aus Metall, Glas oder sonstigem hartem scharfkantigen Material bestehen.

Die obere Folie 2 oder die untere Folie 3 kann eine Kunststoff- oder Aluminiumfolie (insbesondere Hartaluminiumfolie) sein, oder ein Laminat, also ein Schichtverbund aus mehreren Folien. Die obere Folie 2 bzw. die Ausstülpung 2.1 kann vorzugsweise transparent ausgeführt sein. Die obere Folie 2 bzw. das Material der Ausstülpung 2.1 ist dabei plastisch verformbar, sodass nach Wegnahme der zusammendrückenden Kraft von der Ausstülpung 2.1 diese im verformten Zustand verbleibt. Bei einer elastischen Ausstülpung 2.1 würde diese nach Wegnahme der Kraft wieder in ihre Ausgangsform zurück kehren und so die Flüssigkeit aus dem Auspressreservoir 6 in das Flüssigkeitsreservoir 5 teilweise zurücksaugen, was für manche Anwendungen eventuell sogar wünschenswert wäre. Bei den Seitenwänden der Ausstülpung 2.1, welche unterhalb des Applikators 1 liegen, kann eine elastische Rückverformung wünschenswert sein, damit die Applikatorspitze 1.1 nach erfolgter Applikation etwas von der Haut 7 wegbewegt wird und somit die scharfen bzw. spitzen Elemente nicht während der gesamten Testzeit in Kontakt mit der Haut 7 sind. Selbiges kann bei plastisch verformbaren Seitenwänden erreicht werden, wenn nach der Applikation durch Ziehen am Applikator 1 dieser etwas von der Haut 7 wegbewegt wird.

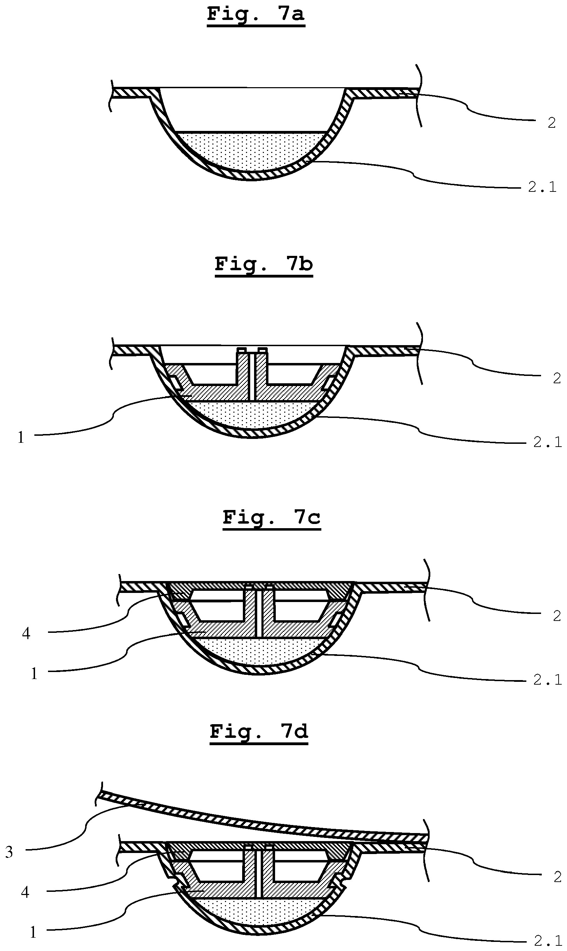

Die Herstellung eines Blisterstreifens kann, wie in den Fig. 7a-7d veranschaulicht, in folgenden Schritten erfolgen:
- Prägen (oder Tiefziehen etc.) der oberen Folie 2, wodurch diese zur Bildung der Blister bleibend verformt wird.
- Wie in Fig. 7a gezeigt, wird die Flüssigkeit in den Blister gegeben, die obere Folie 2 weist dabei mit der Unterseite nach oben, sodass der Blister eine Mulde bildet.
- Wie in Fig. 7b gezeigt, wird der Applikatoren 1 in die Blister eingesetzt.
- Wie in Fig. 7c gezeigt, wird die Öffnung 2.1 bzw. der Blister mit dem Dichtungspfropfen 4 verschlossen. Alternativ können der Applikator 1 und der Dichtungspfropfen 4 gemeinsam eingesetzt werden.
- Wie in Fig. 7d gezeigt, wird der Blisterstreifen mit der unteren Folie 3 versehen, welche den Dichtungspfropfen 4 und die obere Folie 2 abdeckt.
- Wie in Fig. 7d weiter gezeigt wird die obere Folie 2 mit der ringförmigen Mantelfläche des Applikators 1.1 verbunden, beispielsweise indem diese gegen den Applikator 1 gepresst wird, dazu kann wie dargestellt die Mantelfläche des Applikators 1 eine Rille oder eine andere Oberflächenstruktur (z.B. mehrere Vertiefungen, oder mehrerer vertikal und/oder horizontal verlaufende Rillen) aufweisen, sodass die obere Folie 2 formschlüssig mit dem Applikator 1 verbunden wird (ohne Klebung oder Verschweißung). Alternativ kann die obere Folie 2 durch kurze Hitze- und Druckeinwirkung mit dem Applikator 1 verschweißt werden. Die Verbindung der oberen Folie 2 mit dem Applikator 1 kann jederzeit nach Einsetzen des Applikators 1 erfolgen.

Um die untere Folie 3 mit der oberen Folie 2 lösbar zu verkleben, kann die obere Folie 2 bereits vor dem Einbringen der Flüssigkeit mit einer Klebeschicht versehen sein, oder beispielsweise erst nachdem der Dichtungspfropfen 4 eingesetzt wurde. Der Dichtungspfropfen 4 kann ebenfalls bereits vor dem Einsetzen eine Klebeschicht, bzw. zumindest einen punktförmigen Klebeauftrag aufweisen, oder auch nach dem Einsetzen mit dieser/m versehen werden. Bevorzugt weist die Klebeschicht des Dichtungspfropfens 4 eine andere Zusammensetzung als die Klebeschicht der oberen Folie 2 auf, sodass die Klebeschicht des Dichtungspfropfens 4 stark an der Klebeschutzschicht der unteren Folie 3 anhaftet.

Alternativ kann die Klebeschicht mit der unteren Folie 3 aufgebracht werden, indem diese im Bereich der Unterseite 2.2 der oberen Folie 2 leicht klebend und somit löslich mit der Klebeschicht verbunden ist. Im Bereich der Dichtungspfropfen 4 ist die untere Folie 2 stark haften und somit unlöslich mit einer Klebeschicht verbunden.

Das Versehen der Unterseite 2.2 der Folie 2 und der Unterseite des Pfropfens 4 mit Klebstoff, erfolgt beispielsweise durch Beschichtung oder Aufkleben einer doppelseitig klebenden Folie. Die doppelseitige Klebefolie ist dabei in Richtung der oberen Folie 2 hochhaftend, in Richtung der Haut hautverträglich und in einem Maß haftend, dass der Test durchgeführt werden kann ohne beim Abziehen große Schmerzen zu verursachen.

Alternativ zum beschriebenen Verfahren kann die Flüssigkeit durch die Öffnung 1.2 des Applikators 1 injiziert werden, nachdem dieser in den Blister eingesetzt und mit dem Blister verklebt wurde. Bevorzugt weist der Applikator in diesem Fall zumindest zwei Öffnung 1.2 auf, sodass durch die zweite Öffnung Luft beim Befüllen entweichen kann.

Nach Aufbringen der unteren Folie 3 sind die Blister und die darin angeordneten Applikatoren 1 steril verpackt und gegen Kontamination geschützt. Vorteilhaft am gegenständlichen Aufbau des Blisterstreifens ist, dass dieser erst unmittelbar vor der Anwendung zu öffnen ist und kein zusätzliches Instrument verwendet werden muss. Der Allergieteststreifen ist daher lager- und transportierbar und kann von ungeschultem Personal und selbst bei hygienisch bedenklichen Umgebungsbedingungen eingesetzt werden, ohne dass erhöhtes Infektionsrisiko für den Untersuchten oder den Applizierenden besteht.

Da der Allergieteststreifen aufgrund des einfachen Aufbaus günstig in Massenproduktion herstellbar ist und äußerst einfach und sicher in der Anwendung ist, eignet er sich hervorragend für Allergie-Schnelltests mit geringem Zeitaufwand für das Fachpersonal oder für die Selbstanwendung.

Der Blisterstreifen weist zumindest einen ein Allergen enthaltenden Blister auf. Zudem kann ein Blister vorhanden sein, welcher die Flüssigkeit ohne Allergen enthält zum Durchführen der Negativkontrolle und/oder ein Blister mit Histamin zur Durchführung der Positivkontrolle.

Zudem kann eine beliebige Anzahl weiterer Blister vorhanden sein, wobei jeder ein zu testendes Allergen enthält. Der Blisterstreifen kann eine Reihe von Blistern aufweisen, oder zwei oder mehr parallele Reihen von Blistern. Bevorzugt weist der Blisterstreifen eine Reihe mit 8 Blistern auf. Beispielsweise kann so mit zwei unterschiedlich bestückten Blisterstreifen eine Person auf 14 Allergene getestet werden (inklusive Positiv- und Negativtest), bei Aufbringung je eines Streifens auf der Innenseite jedes Unterarms. Die Bestückung der Blisterstreifen kann an das jeweilige Einsatzgebiet (z.B. Geografisch, oder Untersuchung zu einzelnen Allergengruppen z.B. Tiere / Bäume / Gräser...) angepasst werden.

Der Blisterstreifen kann mit einem Klebestreifen kombiniert sein, welcher nach Abziehen des Blisterstreifens auf der Haut verbleibt und die jeweilige Kennzeichnung der im Blister enthaltenen Substanz trägt, wie in Fig. 6 gezeigt. Die Kennzeichnung kann auch am Blisterstreifen selbst angebracht sein und beispielsweise händisch übertragen werden. Eine weitere Möglichkeit der Zuordnung wäre zwei oder mehr Blister in einem charakteristischen Abstand zueinander anzuordnen, damit aus der Position des beispielsweise erhöhten Abstands die Lage des Blisterstreifens auf der Haut nach Abziehen rekonstruiert werden kann, oder eine Schablone nur auf eine Weise und somit eindeutig aufgelegt werden kann. Eine weitere Möglichkeit wäre einen oder mehrere Blister versetzt zu den anderen in Reihe angeordneten Blistern anzuordnen.

Es soll noch beispielhaft und keinesfalls abschließend auf folgende mögliche Verallgemeinerungen gegenüber der bevorzugten, in der Figurenbeschreibung dargelegten Ausgestaltung der Erfindung hingewiesen werden, welche erfindungsgemäß vom gegenständlichen Schutzumfang umfasst sein sollen.

Der Blister bzw. die Ausstülpung der oberen Folie kann eine von der Kuppel abweichende Form haben, beispielsweise zylindrisch oder rechteckig sein, oder ein komplexes Volumen aufweisen, welches beispielsweise aus einem Hohlraum mit zwei oder mehr Kuppeln besteht.

Anstelle des Dichtungspfropfens 4 oder zusätzlich kann der Applikator 1 eine dünne Membran aufweisen, welche die Öffnung 1.2 verschließt und bei Ausüben von Druck reißt.

Denkbar ist es auch dass der Applikator den Blister in drei oder mehr Teilbereiche trennt, welche gegeneinander dicht sind und erst bei Applikation eine Verbindung geöffnet wird. Beispielsweise kann so in einem Teilbereich das Allergen oder ein Wirkstoff als Feststoff vorhanden sein und erst direkt bei der Applikation in der Flüssigkeit aus einem anderen Teilbereich gelöst werden. Das im ersten Teilbereich also dem Flüssigkeitsreservoir 5 enthaltene Medium kann neben Flüssigkeit auch ein Gel, Fett oder Vaseline sein.

## Patentansprüche

1. Blisterstreifen welcher auf die Haut aufklebbar ist und aus zumindest zwei Folien gebildet ist und zumindest einen Applikator aufweist zum Applizieren eines im Blister enthaltenen Mediums und der im Medium enthaltenen Stoffe auf oder in die Haut, wobei der Blisterstreifen eine obere Folie (2) und eine untere Folie (3) umfasst,
wobei die obere Folie (2) mit zumindest einer Ausstülpung (2.1) versehen ist, wobei die die Ausstülpung (2.1) umgebende Unterseite (2.2) der oberen Folie (2) klebend ausgeführt ist und wobei die untere Folie (3) die untere Fläche des Blisterstreifens abdeckt und von der klebenden Unterseite (2.2) der oberen Folie (2) abgezogen werden kann,
wobei ein Applikator (1) in die Ausstülpung (2.1) der oberen Folie (2) eingesetzt ist, welcher die Ausstülpung (2.1) in zumindest zwei Teilvolumen trennt,
wobei der Applikator (1) zumindest eine Öffnung (1.2) aufweist, welche zwei Teilvolumen verbindet,
wobei ein Teilvolumen zwischen dem Applikator (1) und der unteren Folie (3) liegt und
wobei zumindest ein Teilvolumen zur Gänze von der Innenfläche der Ausstülpung (2.1) und dem Applikator (1) umschlossen ist und das Medium enthält.

2. Blisterstreifen nach Anspruch 1, **dadurch gekennzeichnet, dass** zumindest eine der Folien ein Laminat ist.

3. Blisterstreifen nach einem der Ansprüche 1 bis 2, **dadurch gekennzeichnet, dass** die Ausstülpung (2.1) kuppelförmig, formstabil und plastisch verformbar ist.

4. Blisterstreifen nach einem der Ansprüche 1 bis 3, **dadurch gekennzeichnet, dass** der Applikator (1) zumindest ein spitzes oder scharfes Element aufweist, welches mit der Haut in Kontakt bringbar ist.

5. Blisterstreifen nach einem der Ansprüche 1 bis 4, **dadurch gekennzeichnet, dass** die untere Folie (3) ein Dichtungspfropfen (4) aufweist, welcher die Öffnung (1.2) des Applikators (1) verschließt, wobei der Dichtungspfropfen (4) mit der unteren Folie (3) verbunden, insbesondere verklebt, ist, oder durch eine Ausstülpung der unteren Folie (3) gebildet ist.

6. Blisterstreifen nach Anspruch 5, **dadurch gekennzeichnet, dass** der Dichtungspfropfen (4) die gesamte Grundfläche der Ausstülpung (2.1) der oberen Folie (2) verschließt.

7. Blisterstreifen nach einem der Ansprüche 1 bis 6, **dadurch gekennzeichnet, dass** der Applikator (1) scheibenförmig ist und ringsum am Rand der Scheibe mit der Innenwand der Ausstülpung (2.1) verbunden ist, wobei der Applikator (1) im Zentrum der Scheibe eine Applikatorspitze (1.1) aufweist, welche von der Scheibe in Richtung der unteren Folie (3) vorspringt, wobei in der Applikatorspitze (1.1) zumindest eine Öffnung (1.2) durch den Applikator (1) verläuft.

8. Blisterstreifen nach Anspruch 7, **dadurch gekennzeichnet, dass** die Applikatorspitze (1.1) eine oder mehrere zentrale Öffnungen (1.2) aufweist, wobei kranzförmig um die Öffnung (1.2) scharfe oder spitze Elemente von der Applikatorspitze (1.1) in Richtung der unteren Folie (3) bzw. der Haut (7) weisen, wobei bevorzugt zumindest ein scharfes oder spitzes Element eine Hohlnadel ist.

9. Blisterstreifen nach einem der Ansprüche 1 bis 8, **dadurch gekennzeichnet, dass** der Applikator (1) mit der oberen Folie (2) verklebt, verschweißt oder verpresst ist, oder der Rand des Applikators (1) strukturiert ausgeführt ist und die obere Folie (2) formschlüssig in diese Struktur eingepresst ist.

10. Blisterstreifen nach einem der Ansprüche 7 bis 9, **dadurch gekennzeichnet, dass** der Randbereich der Scheibe des Applikators (1) breiter ausgeführt ist als der Bereich der Scheibe zwischen dem Rand und der Applikatorspitze (1.1).

11. Blisterstreifen nach einem der Ansprüche 7 bis 10, **dadurch gekennzeichnet, dass** der Applikator (1) so in die Ausstülpung (2.1) eingesetzt ist, dass der untere Rand der Scheibe beabstandet zur klebenden Unterseite (2.2) der oberen Folie (2) liegt.

12. Blisterstreifen nach einem der Ansprüche 1 bis 11, **dadurch gekennzeichnet, dass** die obere Folie (2) aus plastisch verformbarem Material besteht.

13. Blisterstreifen nach einem der Ansprüche 1 bis 12, **dadurch gekennzeichnet, dass** der Applikator (1) ein formstabiler Körper ist und insbesondere aus Hartplastik gebildet ist.

14. Blisterstreifen nach einem der Ansprüche 1 bis 13, **dadurch gekennzeichnet, dass** eine Entlüftungsöffnung in jenem Teilvolumen der Ausstülpung (2.1) vorhanden ist, welche nicht das Medium enthält, wobei die Entlüftungsöffnung in die Umgebung führt und verschließbar ist, oder in ein abgeschlossenes ausdehnbares Volumen mündet.

15. Blisterstreifen nach einem der Ansprüche 1 bis 14, **dadurch gekennzeichnet, dass** die obere Folie (2) mit mehreren zueinander beabstandeten Ausstülpungen (2.1) versehen ist, wobei die die jeweilige Ausstülpung (2.1) umgebende Unterseite (2.2) der oberen Folie (2) klebend ausgeführt ist,
wobei je ein Applikator (1) in je einer der Ausstülpungen (2.1) der oberen Folie (2) eingesetzt ist, welcher die jeweilige Ausstülpung (2.1) in zumindest zwei Teilvolumen trennt,
wobei jeder Applikator (1) zumindest eine Öffnung (1.2) aufweist, welche zwei Teilvolumen der jeweiligen Ausstülpung (2.1) verbindet,
wobei ein Teilvolumen der jeweiligen Ausstülpung (2.1) zwischen dem Applikator (1) und der unteren Folie (3) liegt und
zumindest ein Teilvolumen zur Gänze von der Innenfläche der jeweiligen Ausstülpung (2.1) und dem jeweiligen Applikator (1) umschlossen ist und das Medium enthält, wobei zumindest das Medium einer Ausstülpung (2.1) ein erstes Allergen enthält,
wobei das Medium der weiteren Ausstülpung (2.1) ausgewählt ist aus der Gruppe von: Medium, welches Histamin enthält; Medium ohne Allergen; Medien mit je zueinander unterschiedlichen Allergenen.

## Claims

1. A blister strip, which can be glued onto the skin and is formed out of at least two sheets and has at least one applicator, for applying a medium contained within the blister and the substances contained in the medium onto or into the skin, wherein the blister strip comprises an upper sheet (2) and a lower sheet (3),
wherein the upper sheet (2) is provided with at least one protrusion (2.1), wherein the underside (2.2) of the upper sheet (2) surrounding the protrusion (2.1) is designed to be adhesive and wherein the lower sheet (3) covers the bottom surface of the blister strip and can be withdrawn from the adhesive underside (2.2) of the upper sheet (2),
wherein an applicator (1), which divides the protrusion (2.1) into at least two partial volumes, is inserted into the protrusion (2.1) of the upper sheet (2),
the applicator (1) has at least one opening (1.2), which connects two partial volumes,
one partial volume is located between the applicator (1) and the lower sheet (3), and
at least one partial volume is entirely enclosed by the inner surface of the protrusion (2.1) and the applicator (1) and contains the medium.

2. The blister strip of claim 1, **characterised in that** at least one of the sheets is a laminate.

3. The blister strip of any one of claims 1 to 2, **characterised in that** the protrusion (2.1) is dome-shaped, dimensionally stable and plastically deformable.

4. The blister strip of any one of claims 1 to 3, **characterised in that** the applicator (1) has at least one acute or sharp element, which can be brought into contact with the skin.

5. The blister strip of any one of claims 1 to 4, **characterised in that** the lower sheet (3) has a sealing plug (4), which occludes the opening (1.2) of the applicator (1), wherein the sealing plug (4) is connected, in particular glued, to the lower sheet (3) or is formed by a protrusion of the lower sheet (3) .

6. The blister strip of claim 5, **characterised in that** the sealing plug (4) occludes the entire base of the protrusion (2.1) of the upper sheet (2).

7. The blister strip of any one of claims 1 to 6, **characterised in that** the applicator (1) is disc-shaped and is connected to the inner wall of the protrusion (2.1) circumferentially at the periphery of the disc, wherein the applicator (1) has at the centre of the disc an applicator tip (1.1), which protrudes toward the lower sheet (3) from the disc, wherein at least one opening (1.2) in the applicator tip (1.1) runs through the applicator (1).

8. The blister strip of claim 7, **characterised in that** the applicator tip (1.1) has one or more central openings (1.2), wherein sharp or acute elements point from the applicator tip (1.1) toward the lower sheet (3) or the skin (7), respectively, annularly around the opening, wherein preferably, at least one sharp or acute element is a hollow needle.

9. The blister strip of any one of claims 1 to 8, **characterised in that** the applicator (1) is glued, welded or pressed to the upper sheet (2), or
the periphery of the applicator (1) is designed to be textured and the upper sheet (2) is positively press-fit into the texture.

10. The blister strip of any one of claims 7 to 9, **characterised in that** the peripheral area of the disc of the applicator (1) is designed to be wider than the area of the disc between the periphery and the applicator tip (1.1).

11. The blister strip of any one of claims 7 to 10, **characterised in that** the applicator (1) is inserted into the protrusion (2.1) in such a manner that the lower periphery of the disc is located spaced apart from the adhesive underside (2.2) of the upper sheet (2).

12. The blister strip of any one of claims 1 to 11, **characterised in that** the upper sheet (2) is made of plastically deformable material.

13. The blister strip of any one of claims 1 to 12, **characterised in that** the applicator (1) is a dimensionally stable body and is in particular formed out of rigid plastic.

14. The blister strip of any one of claims 1 to 13, **characterised in that** a venting opening is present **in that** partial volume of the protrusion (2.1) which does not contain the medium, wherein the venting opening leads into the surrounding area and can be occluded or opens into a sealed, expansible volume.

15. The blister strip of any one of claims 1 to 14, **characterised in that**
the upper sheet (2) is provided with several protrusions (2.1) that are spaced apart from one another, wherein the underside (2.2) of the upper sheet (2) surrounding each respective protrusion (2.1) is designed to be adhesive,
wherein one applicator (1), is inserted in each one of the protrusions (2.1) of the upper sheet (2), dividing the respective protrusion (2.1) into at least two partial volumes,
wherein each applicator (1) has at least one opening (1.2), which connects two partial volumes of the respective protrusion (2.1),
wherein one partial volume of the respective protrusion (2.1) is located between the applicator (1) and the lower sheet (3), and
at least one partial volume is entirely enclosed by the inner surface of the respective protrusion (2.1) and the respective applicator (1) and contains the medium, wherein at least the medium of one protrusion (2.1) contains a first allergen,
wherein the medium of the further protrusion (2.1) is selected from the group of: medium containing histamine; medium without allergen; media each having different allergens as compared the others.

## Revendications

1. Blister qui peut être collée sur la peau et qui est formé d'au moins deux films et comporte au moins un applicateur pour appliquer un milieu contenu dans le blister et les substances contenues dans ledit milieu sur ou dans la peau, ledit blister comprenant un film supérieur (2) et un film inférieur (3), ledit film supérieur (2) étant pourvu d'au moins une protubérance (2.1), la face inférieure (2.2) du film supérieure (2) qui entoure ladite protubérance (2.1) étant réalisée d'une façon adhésive, et le film inférieur (3) recouvrant la surface inférieure du blister et pouvant être décollé de la face inférieure adhésive (2.2) du film supérieur (2),
un applicateur (1) étant inséré dans la protubérance (2.1) du film supérieur (2), ledit applicateur (1) séparant la protubérance (2.1) en au moins deux volumes partiels,
ledit applicateur (1) possédant au moins une ouverture (1.2) qui relie deux volumes partiels,
un volume partiel se trouvant entre l'applicateur (1) et le film inférieur (3) et
au moins un volume partiel étant complètement entouré par la surface intérieure de la protubérance (2.1) et par l'applicateur (1) et contenant le milieu.

2. Blister selon la revendication 1, **caractérisé en ce qu'**au moins un des films est un aggloméré laminé.

3. Blister selon l'une des revendications 1 à 2, **caractérisé en ce que** ladite protubérance (2.1) est en forme de dôme, stable de forme et élastiquement déformable.

4. Blister selon l'une des revendications 1 à 3, **caractérisé en ce que** l'applicateur (1) comprend au moins un élément pointu ou tranchant qui peut être mis en contact avec la peau.

5. Blister selon l'une des revendications 1 à 4, **caractérisé en ce que** le film inférieur (3) présente un bouchon d'étanchéité (4) qui obture l'ouverture (1.2) de l'applicateur (1), ledit bouchon d'étanchéité (4) étant relié, notamment collé, au film inférieur (3) ou est formé par une protubérance du film inférieur (3).

6. Blister selon la revendication 5, **caractérisé en ce que** le bouchon d'étanchéité (4) obture toute la surface de base de la protubérance (2.1) du film supérieur (2).

7. Blister selon l'une des revendications 1 à 6, **caractérisé en ce que** l'applicateur (1) est en forme de disque et est relié tout autour du bord du disque à la paroi intérieure de la protubérance (2.1), l'applicateur (1) ayant une pointe d'applicateur (1.1) au centre du disque, ladite pointe faisant saillie du disque en direction du film inférieur (3), au moins une ouverture (1.2) s'étendant le long de l'applicateur (1) dans la pointe d'applicateur (1.1).

8. Blister selon la revendication 7, **caractérisé en ce que** la pointe de l'applicateur (1.1) présente une ou plusieurs ouvertures centrales (1.2), des éléments pointus ou tranchants arrangés en forme de couronne autour de l'ouverture (1.2) pointant depuis la pointe de l'applicateur (1.1) en direction du film inférieur (3) ou de la peau (7), au moins un élément pointu ou tranchant étant, de préférence, une aiguille creuse.

9. Blister selon l'une des revendications 1 à 8, **caractérisé en ce que** l'applicateur (1) est collé, soudé ou pressé sur le film supérieur (2) ou le bord de l'applicateur (1) a un dessin structuré et le film supérieur (2) est pressée dans cette structure sans aucun espace.

10. Blister selon l'une des revendications 7 à 9, **caractérisé en ce que** la zone de bord du disque de l'applicateur (1) est plus large que la zone du disque entre le bord et la pointe de l'applicateur (1.1).

11. Blister selon l'une des revendications 7 à 10, **caractérisé en ce que** l'applicateur (1) est inséré dans la protubérance (2.1) de telle manière que le bord inférieur du disque soit espacé de la face inféurieure adhésive (2.2) du film supérieur (2).

12. Blister selon l'une des revendications 1 à 11, **caractérisé en ce que** le film supérieur (2) est constitué d'un matériau élastiquement déformable.

13. Blister selon l'une des revendications 1 à 12, **caractérisé en ce que** l'applicateur (1) est un corps stable de forme et est constitué de plastique dur.

14. Blister selon l'une des revendications 1 à 13, **caractérisé en ce qu'**une ouverture de ventilation est présente dans le volume partiel de la protubérance (2.1) qui ne contient pas le milieu, l'ouverture de ventilation menant à l'environnement et pouvant être fermée, ou s'ouvrant dans un volume expansible scellé.

15. Blister selon l'une des revendications 1 à 14, **caractérisé en ce que**
le film supérieur (2) est pourvu d'une pluralité de protubérances (2.1) espacées les unes des autres, la face inférieure (2.2) du film supérieur (2) qui entoure la protubérance respective (2.1) étant réalisée d'une façon adhésive,
un applicateur (1) étant inséré dans chacune des protubérances (2.1) du film supérieur (2), ledit applicateur (1) séparant la protubérance respective (2.1) en au moins deux volumes partiels,
ledit applicateur (1) possédant au moins une ouverture (1.2) qui relie deux volumes partiels de la protubérance respective (2.1),
un volume partiel de la protubérance respective (2.1) se trouvant entre l'applicateur (1) et le film inférieur (3) et
au moins un volume partiel étant complètement entouré par la surface intérieure de la protubérance respective (2.1) et par l'applicateur respectif (1) et contenant le milieu, au moins le milieu d'une protubérance (2.1) contenant un premier allergène,
le milieu de l'autre protubérance (2.1) étant choisi parmi le groupe constitué par un milieu contenant de l'histamine; un milieu sans allergène; un milieu avec différents allergènes.
